# EUROPEAN PATENT APPLICATION

(11) **EP 2 060 626 A1**
(43) Date of publication of application: **20.05.2009**
(21) Application number: 07022260.9
(22) Date of filing: 16.11.2007
(51) Int. Cl.: C12N 5/06, G01N 33/50

(54) **An immortalized human CD4-positive cell and its use for determining the phenotype of a human immunodeficiency virus type 1**

(71) Applicant: Paul-Ehrlich-Institut, 63225 Langen (DE)
(72) Inventor: The designation of the inventor has not yet been filed
(74) Representative: Nordemann, Jan Bernd

(57) **Abstract**

The invention relates to an immortalized CD4-positive cell. According to the invention, this cell comprises a gene encoding a CXCR4 chemokine receptor that is capable of serving as a coreceptor for entry of a Human Immunodeficiency Virus Type 1 (HIV-1) into the cell, and a gene encoding a CCR5 receptor that is not capable of serving as a coreceptor for entry of a Human Immunodeficiency Virus Type 1 into the cell. The invention furthermore relates to a method for the phenotypical characterization of a HIV-1, comprising providing such a cell, adding an HIV-1 to the cell under conditions that allow for the entrance of the HIV-1 into the cell and the replication of the virus in the cell, and measuring the replication of the HIV-1 in the cell.

SEQUENCE LISTING

<110>  Paul-Ehrlich-Institut Bundesamt für Sera und Impfstoffe

<120>  An immortalized human CD4-positive Cell and its use for
       determining the phenotype of a Human Immunodeficiency Virus Type
       1

<130>  FB19108

<160>  18

<170>  PatentIn version 3.3

<210>  1
<211>  19
<212>  DNA
<213>  Artificial

<220>
<223>  Primer CCR5-759 (+)

<400>  1
cttcattaca cctgcagct                                                    19


<210>  2
<211>  21
<212>  DNA
<213>  Artificial

<220>
<223>  Primer CCR5-941 (-)

<400>  2
accagccctg tgcctcttct t                                                 21


<210>  3
<211>  25
<212>  DNA
<213>  Artificial

<220>
<223>  Primer HTLV-I-LTR-72 (+)

<400>  3
cagaagtctg aaaaggtcag ggccc                                             25


<210>  4
<211>  25
<212>  DNA
<213>  Artificial

<220>
<223>  Primer HTLV-I-LTR-607 (-)

<400>  4

gacgtagagt tgagcaagca gggtc                                                25

<210>  5
<211>  35
<212>  PRT
<213>  Human immunodeficiency virus type 1

<400>  5

Cys Thr Arg Pro Ser Asn Asn Thr Ile Thr Gly Ile His Ile Gly Pro
1               5                   10                  15

Gly Arg Met Phe Tyr Arg Thr Gly Ala Ile Ser Gly Asp Ile Arg Lys
            20                  25                  30

Ala Tyr Cys
        35

<210>  6
<211>  35
<212>  PRT
<213>  Human immunodeficiency virus type 1

<400>  6

Cys Thr Arg Pro Gly Asn Asn Thr Arg Lys Ser Ile Pro Ile Gly Pro
1               5                   10                  15

Gly Arg Ala Phe Tyr Ala Thr Gly Asp Val Ile Gly Asp Ile Arg Gln
            20                  25                  30

Ala His Cys
        35

<210>  7
<211>  35
<212>  PRT
<213>  Human immunodeficiency virus type 1

<400>  7

Cys Thr Arg Pro Asn Asn Asn Thr Arg Lys Ser Ile His Ile Gly Pro
1               5                   10                  15

Gly Arg Ala Val Ile Ala Thr Asp Lys Ile Ile Gly Asp Ile Arg Arg
            20                  25                  30

Ala His Cys
        35

<210>  8
<211>  35
<212>  PRT
<213>  Human immunodeficiency virus type 1

<400>  8

Cys Thr Arg Pro Asn Asn Asn Thr Arg Lys Ser Ile His Ile Gly Pro
1               5                   10                  15

Gly Arg Ala Val Ile Ala Thr Glu Lys Ile Ile Gly Asp Ile Arg Arg
            20                  25                  30

Ala His Cys
        35

<210>  9
<211>  35
<212>  PRT
<213>  Human immunodeficiency virus type 1

<400>  9

Cys Thr Arg Pro Asn Asn Asn Thr Arg Lys Ser Ile His Ile Gly Pro
1               5                   10                  15

Gly Arg Ala Phe Tyr Ala Thr Lys Gly Ile Ile Gly Asp Ile Arg Arg
            20                  25                  30

Ala His Cys
        35

<210>  10
<211>  35
<212>  PRT
<213>  Human immunodeficiency virus type 1

<400>  10

Cys Thr Arg Pro Asn Asn Asn Thr Arg Lys Ser Ile His Ile Gly Pro
1               5                   10                  15

Gly Arg Ala Phe Tyr Ala Thr Gly Asp Ile Ile Gly Asn Ile Arg Arg
            20                  25                  30

Ala His Cys
        35

<210>  11

<211>  34
<212>  PRT
<213>  Human immunodeficiency virus type 1

<400>  11

Cys Thr Arg Pro Asn Asn Lys Lys Ile Arg His Ile His Ile Gly Pro
1               5                   10                  15

Gly Arg Ala Phe Tyr Gly Thr Asp Ile Thr Gly Asp Ile Arg Gln Ala
            20                  25                  30

His Cys

<210>  12
<211>  34
<212>  PRT
<213>  Human immunodeficiency virus type 1

<400>  12

Cys Thr Arg Pro Asn Asn Asn Thr Arg Arg Ser Ile His Ile Gly Pro
1               5                   10                  15

Gly Arg Ala Phe Tyr Gly Thr Asp Ile Ile Gly Asp Ile Arg Gln Ala
            20                  25                  30

His Cys

<210>  13
<211>  34
<212>  PRT
<213>  Human immunodeficiency virus type 1

<400>  13

Cys Thr Arg Pro Asn Asn Lys Arg Ile Arg His Ile His Ile Gly Pro
1               5                   10                  15

Gly Arg Ala Phe Tyr Gly Thr Asp Ile Thr Gly Asp Ile Arg Gln Ala
            20                  25                  30

His Cys

<210>  14
<211>  36
<212>  PRT

<213>  Human immunodeficiency virus type 1

<400>  14

Cys Thr Arg Pro Asn Asn Asn Ile Ile Arg Arg Arg Ile His Ile Gly
1               5                   10                  15

Pro Gly Arg Ala Phe Tyr Ala Thr Arg Gly Ile Thr Gly Asn Val Arg
            20                  25                  30

Lys Ala Tyr Cys
        35

<210>  15
<211>  35
<212>  PRT
<213>  Human immunodeficiency virus type 1

<400>  15

Cys Thr Arg Pro Asn Asn Asn Thr Arg Arg Gly Ile His Ile Gly Pro
1               5                   10                  15

Gly Ser Ala Phe Tyr Thr Thr Gly Gln Ile Ile Gly Asp Ile Arg Lys
            20                  25                  30

Ala His Cys
        35

<210>  16
<211>  35
<212>  PRT
<213>  Human immunodeficiency virus type 1

<400>  16

Cys Thr Arg Pro Asn Asn Asn Thr Arg Arg Gly Val His Ile Gly Pro
1               5                   10                  15

Gly Ser Ala Phe Tyr Thr Thr Gly Gln Ile Ile Gly Asp Ile Arg Lys
            20                  25                  30

Ala His Cys
        35

<210>  17
<211>  35
<212>  PRT
<213>  Human immunodeficiency virus type 1

<400>  17

Cys Thr Arg Pro Asn Asn Asn Thr Arg Arg Ser Ile His Ile Ala Pro
1               5                   10                  15

Gly Ser Thr Phe Tyr Ala Thr Gly Gln Ile Ile Gly Asp Ile Arg Gln
            20                  25                  30

Ala His Cys
        35

<210>  18
<211>  35
<212>  PRT
<213>  Human immunodeficiency virus type 1

<400>  18

Cys Thr Arg Leu Ser Asn Asn Thr Arg Lys Ser Ile Thr Leu Gly Pro
1               5                   10                  15

Arg Ala Thr Phe Tyr Ala Thr Gly Glu Val Ile Gly Asp Ile Arg Gln
            20                  25                  30

Ala His Cys
        35

## Description

The present invention relates to an immortalized CD4-positive cell, its use and to a method for producing such a cell. Furthermore, the invention relates to a method and a kit for the phenotypical characterization of a human immunodeficiency virus type I (HIV-1).

For entry into target cells Human Immunodeficiency Virus Type 1 (HIV-1) uses the chemokine receptors CCR5 and CXCR4 as coreceptors in addition to CD4. Coreceptor specificity often shows a characteristic pattern of evolution during the course of infection with HIV-1 strains responsible for transmission and predominating during the long asymptomatic phase of infection being restricted to CCR5 usage (R5-tropic) (Berger et al., 1999). These replicate efficiently in memory cells, which express high levels of CCR5 and are particularly abundant in the gut-associated lymphoid tissue, the site of primary HIV replication (Brenchley et al., 2004). In more than half of the infected individuals, HIV strains emerge that use CXCR4 (X4-tropic), a homeostatic chemokine receptor expressed on a broader range of cells (Connor et al., 1997; Scarlatti et al., 1997; Delobel et al., 2005). This phenomenon occurs later in the course of infection concomitant with immunologic and clinical signs indicating disease progression. Such a switch in coreceptor usage from R5 to X4 is common in clade B HIV-1 infections but less common in clade C infections (Cecilia et al., 2000; Johnston et al., 2003). Evidence from ex vivo studies and experiments in animal models indicate that the presence of X4 viruses causes the acceleration of CD4+ T-cell depletion (Penn et al., 1999; Feinberg et al., 2002) and increased pathogenicity through CXCR4-mediated killing of uninfected CD4 T cells (Jekle et al., 2003). The long delay between primary infection and the emergence of X4 viruses has been explained by the diminished replication fitness of the switch variants, by a decreased efficiency in coreceptor use and by unique mutational pathways (Pastore et al., 2004). Switch in coreceptor use has also been described for patients undergoing antiretroviral therapy (Holtkamp et al., 2000; Kitchen et al., 2004; Delobel et al., 2005).

The primary determinant of coreceptor usage is the third variable (V3) region of the HIV envelope glycoprotein (Cheng-Mayer et al., 1990; Fouchier et al., 1992; Shioda et al., 1992; Chesebro et al., 1996; Hung et al., 1999; Hoffmann et al., 2002). As knowledge of the viral phenotype is often critical in both the research and clinical settings, sequencing-based methods to predict phenotypes have been developed, based on the V3 genotypes of characterized HIV isolates (Briggs et al., 2000; Resch et al., 2001; Jensen et al., 2003; Pillai et al., 2003). In addition to the V3 region, sequence changes within the V1, V2 and C4 regions of gp120 env have also been shown to have a profound impact on coreceptor use (reviewed in Hartley et al., 2005). Many investigators determine coreceptor use by infecting indicator cell lines genetically engineered to express CD4 plus one of the chemokine receptors (Vodicka et al., 1997; Vodros and Fenyo, 2005). Obvious limitations to this type of biological phenotyping include the complex balance between the level of CD4 expression and that of the coreceptor of interest plus the background expression of other (un)known coreceptors. To facilitate the determination of coreceptor preference in assays more relevant to the situation in vivo, coreceptor usage studies have been performed using PBMC from individuals homozygous for the CCR5-Δ32 allele encoding a severely truncated protein that fails to reach the cell surface (Xiao et al., 1998b; Holtkamp et al., 2000; Meister et al., 2001).

However, such assays are time-consuming, restricted in the number of samples that can be tested, and depend on the availability of a suitable blood donor.

### Description of the Invention

Accordingly, the problem underlying the present invention was to provide a means of determining which coreceptor a given viral isolate of the Human Immunodeficiency Virus Type 1 uses to enter a CD4 positive T cell.

This problem is solved by an immortalized human CD4-positive T cell according to the present invention. Such a cell comprises a gene encoding a CXCR4 chemokine receptor that is capable of serving as a coreceptor for entry of a Human Immunodeficiency Virus Type 1 into the cell, and a gene encoding a CCR5 receptor that is not capable of serving as a coreceptor for entry of a Human Immunodeficiency Virus Type 1 into the cell.

Such a cell is resistant to infection with CCR5-tropic HIV viruses, but is highly susceptible to infection with CXCR4-tropic isolates. This cell is therefore suitable for discriminating between viruses using CCR5 and CXCR4 as co-receptors.

Both the CXCR4 chemokine receptor and the CCR5 receptor are expressed on the surface of the cell and can be recognized by a Human Immunodeficiency Virus Type 1 as a coreceptor of HIV-1 together with the CD4 receptor when entering a cell.

It will be understood by a person of skill in the art that in order to achieve this technical result, in case of the cell of the invention being diploid or polyploid, the cell does not contain an allele that encodes for a CCR5 receptor that is capable of serving as a coreceptor for HIV-1 entry.

Also, the cell of the invention does not harbor a wild type gene that is merely not being transcribed, but rather contains a mutant version of the CCR5 gene, which renders the CCR5 receptor non-functional with respect to HIV-1 entrance into the cell. Thereby, it can be excluded that the expression of a (so far) untranscribed wild type gene might be induced by a stimulus (unintentionally) provided to the cell in culture. In principle, it is also possible to e.g. remove the promoter of the CCR5 gene. This approach, however, might not lead to the complete abolishment of transcription of the CCR5 gene. Therefore, the presence of a mutated CCR5 gene in the cell is preferred, rendering the CCR5 receptor incapable of mediating HIV-1 entrance into the cell.

The term "capable of serving as a coreceptor" refers to a surface protein that allows an HIV-1 (of any subtype) to enter the cell through interaction between this surface protein of the cell and the surface protein gp120 env of the virus. Ways of determining virus entrance are known to a person of skill in the art.

The CCR5 receptor can be present in several possible forms that render the CCR5 receptor incapable of serving as a coreceptor for HIV-1 entry. It is possible, for example, that the gene encoding the CCR5 receptor bears a mutation or deletion in a part of the CCR5 gene that encodes for a functional important domain with respect to HIV-1 entrance. A person of skill in the art is able to determine such parts of the gene. In one preferred embodiment of the invention, the gene encoding the CCR5 receptor bears a Δ32 deletion. This type of deletion is described in detail e.g. in Dean et al., 1996, Liu et al., 1996, and Samson et al., 1996.

In another preferred embodiment of the cell, it is dependent on interleukin-2 (IL-2) for growth.

In another preferred embodiment, the cell is positive for at least one of the surface markers CD25 (IL-2 receptor α chain), CD45RO and/or HLA-DR. The presence of at least one of these markers indicates that the cell is an activated T cell. It is preferred that the cell of the invention is positive for all three markers, CD25, CD45RO and HLA-DR.

In a further preferred embodiment, the cell is diploid. This differentiates the cell of the invention from most available cell lines, which are usually polyploid. A diploid cell resembles the genetic state of human body cells (save sperm and egg cells) and therefore is a good model system for analyzing an HIV-1. The cell of the invention therefore is usually a human cell, but could also be a cell e.g. from a monkey or ape.

For a diploid cell, a person of skill in the art will recognize that both alleles of the CCR5 receptor need to encode for non-functional receptors that do not allow for an HIV-1 to enter the cell using the CCR5 receptor as a coreceptor. Accordingly, it is preferred, for example, that the diploid cell is homozygous for the CCR5 Δ32 allele (Δ32/Δ32).

The cell of the invention as described above can be, in a preferred embodiment, a cell from a clonal cell line. The disposal of a clonal cell line facilitates conducting experiments, because a virtually unlimited amount of cells with an identical genetic background can be made available. This is of particular usefulness with regard to an assay that allows for the determination of the phenotype of an HIV-1 strain. It is particularly preferred that the cell of the invention is a cell that has been deposited according to the Budapest Treaty at the European Collection of Cell Cultures (ECACC), Health Protection Agency - Porton Down, Salisbury, Wiltshire SP4 0JG, UK, under the name of IsnoR5 clone 1 (provisional accession number 07111401). The cell according to the invention can also be part of a tissue or an organism.

A preferred embodiment of a cell line according to the invention is a cell line as referred to as IsnoR5 cell line (clones 1 and clone 2) as described in the examples.

The problem underlying the present invention is also solved by the use of an immortalized CD4-positive T cell such as described above. According to the invention, a chemokine receptor CCR5-negative T cell (or cell line) can be used for determining the phenotype of a Human Immunodeficiency Virus Type 1 (HIV-1). This is done by testing whether such a cell can be entered by a Human Immunodeficiency Virus Type 1. Infection of the cell then indicated that the HIV-1 must have entered by interacting with a coreceptor other than CCR5, e.g. by interacting with CXCR4.

As will be described later, such a cell can also be used together with a CXCR4 specific antagonist, for example bicyclam AMD 3100, to determine whether an HIV-1 uses CXCR4 as a coreceptor or an alternative coreceptor (i.e. a coreceptor alternative to CCR5 and CXCR4) for entry into the target T cell.

The problem underlying the present invention is also solved by a method for producing an immortalized human CD4-positive T cell (or an immortalized T cell line) for the phenotypical characterization of a human immunodeficiency virus type I. This method will result in a cell as described above. According to the invention, the method comprises:
Firstly, peripheral blood mononuclear cells (PBMC) from a healthy donor, who is expressing a gene encoding a CCR5 receptor that is not capable of serving as a coreceptor for entry of a Human Immunodeficiency Virus Type 1 into the cell, are obtained.
Approximately 1 % of the Caucasian human population is homozygous for a 32 base pair (bp) deletion in the CCR5 gene. These individuals produce a non-functional protein and are highly resistant to HIV-1 infection, demonstrating the pivotal role of CCR5 for viral transmission (Liu et al., 1996; Samson et al., 1996). One way of practicing the method according to the invention is to isolate peripheral blood mononuclear cells (PBMC) from a healthy human donor, who is expressing a gene encoding a CCR5 receptor that is not capable of serving as a coreceptor for entry of a Human Immunodeficiency Virus Type 1 into the cell. A person of skill in the art will be able to determine which individuals will qualify for this method, in particular with reference to the examples provided herein. Alternatively, such a cell can also be obtained by introducing adequate mutations in the CCR5 gene in the genome of a (wild type) cell using standard molecular biology procedures. Preferably, the donor is homozygous for the CCR Δ32 allele.
Secondly, those cells from the peripheral blood mononuclear cells are isolated that are CD4-positive cells.
The isolation step of the present method can be performed principally in two different ways. For one, it is possible to deplete the CD4-positive cells from the population of PBMC using anti-CD8 antibodies. In a preferred embodiment, the antibodies are labeled with a means for separating the cells, such as a magnetic bead. Then, the CD4-positive cells can be separated using a magnetic cell separation procedure, as known in the art.
Alternatively, it is possible to select the cells from the PBMC that are CD4-positive, by means of enrichment with an anti-CD4 antibody, while the CD4-negative cells are washed away. As described above, the antibody can be labeled with a means for separating the cells, such as a magnetic bead.
Thirdly, an isolated CD4-positive cell is immortalized. From this immortalized isolated CD4-positive cell, a clonal cell line can be established using methods known in the art.
Several methods are known for immortalizing a cell. This includes infection of the cell to be immortalized with a HTLV-1 (through HTLV-I transformation). Immortalization using a transforming gene of HTLV-1 or transfection of the human telomerase gene is particularly preferred, since the resulting cell is not potentially infectious. Details of how to perform such procedures are known to a person of skill in the art.

This assay allows for the quantitative evaluation of HIV coreceptor use based on a CD4-positive T cell line established from a donor homozygous negative for CCR5 (CCR5⁻/CCR5⁻). It allows the diagnostic evaluation whether a viral isolate uses CCR5, CXCR4 or an alternative receptor as a coreceptor for viral entrance into a target T cell.

The problem underlying the present invention is also solved by a method for the phenotypical characterization of a human immunodeficiency virus type I. According to the invention, the following steps are performed:
Firstly, a cell (or a cell line) is provided that is expressing either a CXCR4 chemokine receptor that is capable of serving as a coreceptor for entry of a Human Immunodeficiency Virus Type 1 into the cell, or a CCR5 receptor that is not capable to mediate entry of a Human Immunodeficiency Virus Type 1 into the cell as a coreceptor. Alternatively, the provided cell expresses no CCR5 receptor, which can be due to the presence of a non-transcribed wild type gene in the cell or an altered or mutated form of the CCR5 receptor gene and/or of the promoter of the CCR5 receptor gene.
Secondly, a Human Immunodeficiency Virus Type 1 is added to the cell (or cell line). This is done under conditions that allow for the infection of the cell by the HIV-1 and for the replication of the virus in the cell.
Thirdly, the replication of the virus in the cell (or cell line) is measured to determine a replication value or the replication rate. This can be done by removing the supernatant from the infected cells, inactivating the HIV-1 through the addition of a suitable detergent and transferring the supernatant to a cell culture container appropriate for performing an enzyme-linked immunosorbent assay (ELISA). The container can be coated e.g. with an antibody that specifically recognizes the viral Gag protein.
Fourthly, the replication value obtained can be compared to the replication of an HIV-1 virus in a reference cell, such as a human peripheral blood lymphocyte (PBL). Virus levels can, e.g., be calculated by comparison with a standard HIV-1 supernatant of known p24 concentration. This fourth step is optionally performed when absolute values for the virus replication need to be obtained.

Such a method is useful for screening patients infected with HIV-1 prior to treatment with CCR5 antagonists.

The cell provided in a preferred embodiment of the method of the invention is a CEM174 cell, which is an established polyploid cell line known not to express CCR5 but to express CXCR4. It is preferred that the cell provided is a cell of the invention as described above, because primary HIV-1 isolates tend not to grow well in CEM174 cells or in other established polyploidy cell lines.

In another preferred embodiment of the present method, the cell is provided together with a CXCR4 specific antagonist, such as bicyclam AMD 3100. HIV-1 isolates able to replicate in cells of the invention in the presence of the selective CXCR4 antagonist AMD 3100 are using a receptor other than CCR5 or CXCR4 (Table I).

The prototype compound of the bicyclams, AMD3100, is a highly specific antagonistic for CXCR4. It binds to a conserved region of CXCR4, preventing infection of cells by CXCR4-tropic virus strains (Schols et al., 1999). AMD3100 is also able to efficiently block dualtropic viruses that use CCR5 and CXCR4 equally well in human PBMC (Schols et al., 1999). Using IsnoR5 clones 1 or 2, a quantitative evaluation of the coreceptor usage of HIV-1 isolates can be performed.

Coreceptor usage is an important feature of the biological phenotype of HIV-1 variants, because it plays a critical role in virus tropism and pathogenesis. A switch in viral coreceptor usage from CCR5 to CXCR4 can be observed in approximately 50 % of infected individuals and this coincides with the rapid depletion of CD4-positive T lymphocytes. However the other 50 % of infected individuals who progress to AIDS harbor CCR5-dependent viral strains only.

There is evidence to suggest that CCR5-using viruses can evolve over time in the infected host to gain an enhanced tropism for macrophages, having an increased ability to utilize the relatively low levels of CD4 and CCR5 expressed on these cells. Furthermore, these late-emerging R5 strains seem to have an increased ability to induce CD4+ T lymphocyte depletion (Gorry et al., 2005). In addition, it has been reported that changes in the cytokine milieu influence chemokine receptor expression on HIV target cells and this may explain the emergence of viruses with different tropisms that facilitate HIV transmission and disease progression (Patterson et al., 1999).

Isolation of viral variants from patients capable of using a broad range of coreceptors including CCR5, CCR3, CCR2b, and CXCR4 is correlated with HIV disease progression. In contrast, long term non progressors (LTNP) carry virus strains that use CCR5 solely as a coreceptor (Connor et al., 1997; Scarlatti et al., 1997; Xiao et al., 1998a). CXCR4 is used almost exclusively by virus strains that emerge during the advanced stages of the infection.

Although prediction of receptor usage is possible by sequencing, the computer algorithms are based on the amino acid sequence of the viral envelope gp120 V3 loop. However, it is known that other areas of the protein, such as the V1 and V2 regions, can strongly influence receptor usage and verification of the actual biological phenotype is far more reliable (Jansson et al., 2001; Masciotra et al., 2002). A cell described above can be used to detect X4-using viruses at limiting amounts and is therefore useful for early diagnosis of recently emerged switches in coreceptor usage of HIV infected individuals.

The measurement of the viral replication is performed preferably using an antigen capture enzyme-linked immunosorbent assay (ELISA) against the HIV-1 Gag p24 core protein.

A method as described above can be used for the diagnosis of a coreceptor change of Human Immunodeficiency Virus Type 1, particularly in an infected individual. Thereby, the method can be used to identify a switch from CCR5 as the coreceptor used to CXCR4 or an alternative coreceptor, that has thus for not been identified.

In an alternative application, the present cell can be used in a method for selecting a CXCR4-using human immunodeficiency virus type I from a population of both CCR5-using human immunodeficiency viruses type 1 and CXCR4-using human immunodeficiency viruses type 1. The method can also be applied when the population contains only a small amount of a CXCR4-using human immunodeficiency virus type I while the population is dominated by CCR5-using human immunodeficiency viruses type 1.

The problem underlying the present invention is also solved by a kit. This kit can be used both for the phenotypical characterization of a human immunodeficiency virus type I and also for selecting a CXCR4-using human immunodeficiency virus type I from a population of CCR5-using and CXCR4-using human immunodeficiency viruses type 1. According to the invention, the kit comprises at least one cell as described above, and a monoclonal or polyclonal antibody that is specific against the HIV-1 Gag p24 core protein. This antibody can be used in an ELISA assay to determine the replication rate of the virus. In addition, the kit may also comprise a CXCR4 specific antagonist, such as bicyclam AMD 3100, to assay entry of the virus via a coreceptor that is neither CCR5 nor CXCR4. The kit may also contain cell culture media, other chemicals, etc.

The invention described herein, in a preferred embodiment, does not concern a process for cloning human beings, processes for modifying the germ line genetic identity of human beings, uses of human embryos for industrial or commercial purposes or processes for modifying the genetic identity of animals which are likely to cause them suffering without any substantial medical benefit to man or animal, and also animals resulting from such processes.

### FIGURES

The invention is now described in a preferred embodiment with reference to the figures.

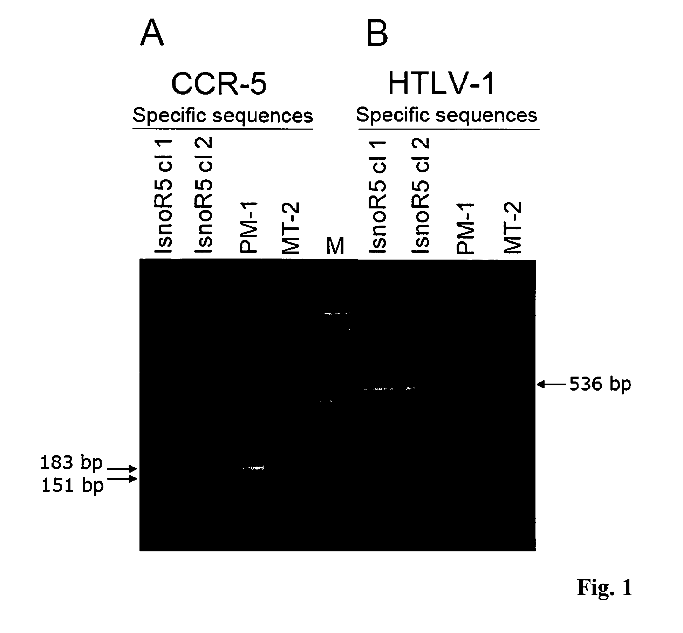

### Figure 1: Genotyping of IsnoR5 cell clones 1 and 2.

(A) To determine CCR5 coreceptor genotypes, chromosomal DNA was PCR amplified using a primer pair flanking the 32 bp deletion in the CCR5 gene. Homozygous wild-type CCR5 genes gave a 183 bp PCR product, whereas genes with the homozygous 32 bp deletion gave one of 151 bp. (B) To detect HTLV-I proviral sequences, a 536 bp fragment of the HTLV-I LTR was amplified. Amplified products were subjected to electrophoresis on a 2.5 % agarose gel and visualized by ethidium bromide staining.

### Figure 2: Immunophenotype of IsnoR5 clones 1 and 2.

Expression of surface markers CD3, CD4 and CD8 on IsnoR5 clone 1 and 2 as analyzed by dual-color FACS. The histograms represent an overlay of cells, stained with mAb against surface markers CD25, HLA-DR, CCR5, or CXCR4 (grey shaded lines), or with the isotype-matched controls (black lines).

### Figure 3: Replication kinetics of the CXCR4-tropic HIV-1 strain SF 2 and CCR5-tropic HIV strain SF 162 in clones 1 and 2 of the CCR5-negative IsnoR5 cell line as well as in PM-1.

Replication was measured by infecting 5 x 10⁵ cells of each cell with 25 TCID₅₀ of HIV-1 SF 2, and HIV-1 SF 162, respectively in triplicate (a, b, c). Cells were cultured and at 0, 3, 5, 7, 10, 12, 14, 17, 19 and 21 days post infection, cell-free supernatants were removed and evaluated by the HIV-1 Gag p24 antigen assay.

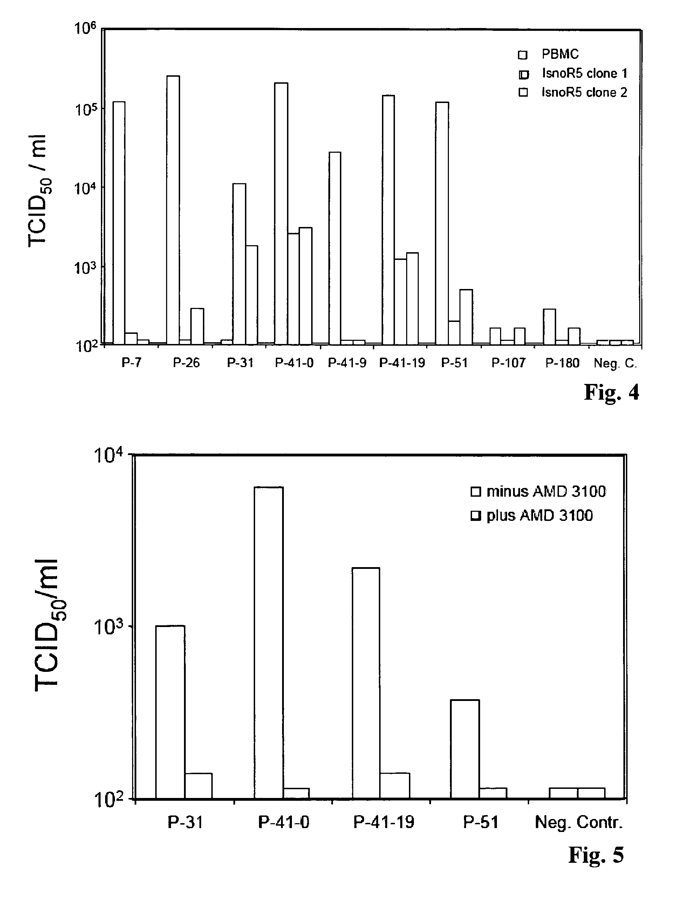

### Figure 4: Experimental determination of coreceptor preference by measuring viral replication in CCR5-negative IsnoR5 clones 1 and 2.

A panel of primary HIV-1 isolates was used to infect IsnoR5 clones 1 and 2 as well as PBMC from a healthy donor. Virus-containing supernatants, normalized to 5 ng/ml HIV-1 Gag p24, were titrated in the different cells which were incubated for 14 days before testing of cell-free supernatants for viral Gag protein by p24 antigen capture assay. The number of positive wells was used to determine the TCID₅₀ of the original sample.

### Figure 5: Experimental determination of coreceptor preference by measuring viral replication in CCR5-negative IsnoR5 clone 2 cells in the presence of the CXCR4 inhibitor AMD 3100.

Those primary HIV-1 isolates able to replicate in IsnoR5 clones 1 and 2 (Figure 4) were used to infect IsnoR5 clone 2 cells in the presence and absence of the bicyclam AMD 3100, a CXCR4-specific inhibitor. Virus-containing supernatants, normalized to 5 ng/ml HIV-1 Gag p24, were titrated in the IsnoR5 clones in the absence and presence of 1 µg/ml AMD 3100. After 14 days, cell free supernatants tested for viral Gag protein by p24 antigen capture assay. The number of positive wells was used to determine the TCID₅₀ of the original sample.

### EXAMPLES

### Materials and Methods

### Patient recruitment and virus isolation

HIV-1-positive patients were recruited from the University Hospital, Frankfurt, Germany. Virus isolation was performed as previously described (Holtkamp et al., 2000) except that viral replication was detected using an antigen capture enzyme-linked immunosorbent assay (ELISA) for the HIV-1 Gag p24 core protein. Inactivated supernatants (0.2 % Tween 20) were titrated on ELISA plates coated with the Gag-specific monoclonal antibody Ag3.0 (hybridoma kindly provided by Jon Allan, Southwest Foundation, San Antonio, Texas, USA). Alternatively, other suited antibodies against HIV-1 proteins can be used. Captured protein was detected using pooled sera from HIV-1 positive individuals followed by a goat antihuman immunoglobulin G-peroxidase conjugate (Sigma-Aldrich, Taufkirchen, Germany). Virus levels were calculated by comparison with a standard HIV-1 supernatant of known p24 Gag concentration.

### Cells

MT-2, an HTLV-I producing cell line (*Miyoshi et al*., 1981) (American Type Culture Collection) and PM-1, a CD4⁺ clone derived from the Hut78 T-cell line (Lusso et al., 1995), were maintained in R10 medium (RPMI 1640 supplemented with 10 % fetal calf serum; Seromed®, Biochrom, Berlin, Germany) and passaged twice weekly.

### Establishment of a CCR5 negative T cell line

Peripheral blood mononuclear cells (PBMC) from a healthy donor homozygous for the CCR5 Δ32 allele were separated from heparinized blood by Ficoll-Histopaque 1077 (Sigma, Steinheim, Germany) density gradient centrifugation and depleted of CD8⁺ cells using anti-CD8 immunomagnetic beads (Miltenyi Biotech, Bergisch Gladbach, Germany) according to the manufacturer's instructions. The remaining cell population was resuspended in RPMI 1640 medium supplemented with 20 % FCS (R20) and stimulated by 2 µg/ml phytohemagglutinin (PHA, Murex Biotech, Dartford, UK) for two days. After washing, cells were resuspended in R20 medium containing 180 U/ml recombinant IL-2 (rIL-2, Proleukin, Chiron, Amsterdam, Netherlands). HTLV-I transformation was achieved by co-culturing the PBMC with gamma-irradiated MT-2 (500 rads; OB29 cesium source, MCP-STS Braunschweig, Germany) at a PBMC:MT2 ratio of 3:1. Cocultures were maintained in the presence of 180 U/ml rIL-2 and passaged as needed. An immortalized cell line was established after 2 months of culture and named IsnoR5.

### Establishment of cell clones

Cell clones were established from immortalized CCR5 negative T cell line IsnoR5 by limiting dilution. Cells were dispensed at 0.8 cells per well into round-bottomed 96-well plates (Nunc, Wiesbaden, Germany) in R10 medium containing 180 U/ml IL-2 and fed weekly. After 4 to 5 weeks arising clones were transferred to 48-well plates. These continued to grow continuously in large clusters. IsnoR5 clones 1 and 2 were used for further investigations.

### Determination of CCR5 coreceptor genotype

The CCR5 genotype of the cells was determined by PCR using 1 µg genomic DNA with primers CCR5-759 (+): 5' -CTTCATTACACCTGCAGCT- 3' (SEQ ID NO 1) and CCR5-941 (-): 5'-ACCAGCCCTGTGCCTCTTCTT- 3' (SEQ ID NO 2) flanking the 32 bp deletion. PCR of DNA from donors with the wild-type CCR5 genotype (CCR5⁺/⁺) gave products of 183 bp, whereas donors with the homozygous deletion (CCR5 Δ32/ Δ32) gave a 151 bp product.

### Detection of HTLV-I provirus

To detect proviral HTLV-I sequences in chromosomal DNA, primers HTLV-I-LTR-72(+): 5'-CAGAAGTCTGAAAAGGTCAGGGCCC (SEQ ID NO 3) and HTLV-I-LTR-607(-): 5'-GACGTAGAGTTGAGCAAGCAGGGTC (SEQ ID NO 4) were used to amplify a 536 bp fragment. Numbers correspond to positions in the HTLV-I genome (Seiki et al., 1983). Amplified products were separated by electrophoresis on 2.5 % agarose gels and visualized by ethidium bromide staining.

### Immunophenotyping

Immunophenotyping was performed by flow cytometry in a FACSCalibur (Beckton Dickinson (BD), Heidelberg, Germany). Briefly, 2 x 10⁵ cells in phosphate-buffered saline (PBS) were stained for surface markers with 10 µl each of the following FITC, PE or PerCP conjugated monoclonal antibodies (mAbs): Leu4 (anti-CD3, BD), Leu3a (anti-CD4, BD), Leu2a (anti-CD8, BD), 2A3 (anti-CD25, BD), *anti*-human HLA-DR (BD/Pharmingen), 12G5 (anti-CD184) (anti-CXCR4, BD/Pharmingen), anti-CD195 (anti-CCR5, BD/Pharmingen) for 20 min in the dark on ice. Isotype-control antibodies (anti-IgG1 and anti-IgG2, respectively) were used to determine background staining and autofluorescence. Following staining, the cells were washed twice with PBS/10 % fetal calf serum and fixed with 1 % paraformaldehyde.

### Sequence analysis of V3 region

Either viral RNA was prepared from the cell-free supernatant of infected cell cultures using QIAamp viral RNA kit (Quiagen, Hilden, Germany) and reverse transcribed using the First strand cDNA Synthesis Kit (Amersham Biosciences, UK) or chromosomal DNA was used as template. PCR amplification of the V3 region was performed as previously described (Holtkamp et al., 2000). Amplification products were ligated into the pGEMT vector (Promega, Mannheim, Germany) and nucleotide sequencing was performed using dye-labeled terminators.

### Infections

To study replication kinetics, 5x10⁵ cells (IsnoR5 clones 1 and 2, PM-1) were incubated with 25 to 100 TCID₅₀ of the different viruses (HIV-1 SF2, HIV-1 SF162) for 1 hour. After washing three times in medium, cells were cultured in 48-well-plates for up to 21 days. Aliquots of the supernatants were removed 0, 3, 5, 7, 10, 12, 14, 17, 19, and 21 days post infection and frozen at -20°C until assayed for p24 content. All samples from one experiment were thawed, inactivated by the addition of Tween 20 to a final concentration of 0.2 % and their HIV-1 Gag protein concentrations measured using the p24 antigen capture assay described above.

### Quantification of infectivity and determination of coreceptor usage of viral isolates

Virus-containing supernatants were serially diluted in media and aliquoted (50 µl per well, six replicates per dilution) into U-well microtiter plates containing 2 x 10⁵ of IsnoR5 clones 1 or 2 or 4.5 x 10⁵ PBMC from healthy blood donors in 150 µl medium. In CXCR4-blocking experiments, cells were preincubated for 1 hour and maintained in 1 µg/ml of the bicyclam CXCR4-antagonist AMD 3100. After 14 days incubation at 37°C, cultures were centrifuged and cell-free supernatants were inactivated by the addition of Tween 20 to a final concentration of 0.2 %. 25 µl of each sample was transferred to the corresponding well of an ELISA plate for detection of viral Gag protein using the p24 antigen capture assay. The number of positive wells was then used to determine the TCID₅₀ of the original sample using the Spearmann-Karber method.

The following reagent was obtained through the NIH AIDS Research and Reference Reagent Program, Division of AIDS, NIAID, NIH: bicyclam JM-2987 (hydrobromide salt of AMD-3100).

### Example 1: Establishment of CCR5-negative CD4-positive T cell line IsnoR5 and clones thereof

Donors homozygous for the CCR5-Δ32 allele (Dean et al., 1996; Liu et al., 1996; Samson et al., 1996) were identified by PCR screening of volunteers. Coculturing of CD8-depleted PBMC from donor IS with gamma-irradiated HTLV-I producer MT-2 cells resulted in the establishment of an immortalized, IL-2 dependent cell line, named IsnoR5. Cell cloning by limiting dilution yielded cell clones, two of which (IsnoR5 clone 1 and 2) have been growing continuously for over 9 months.

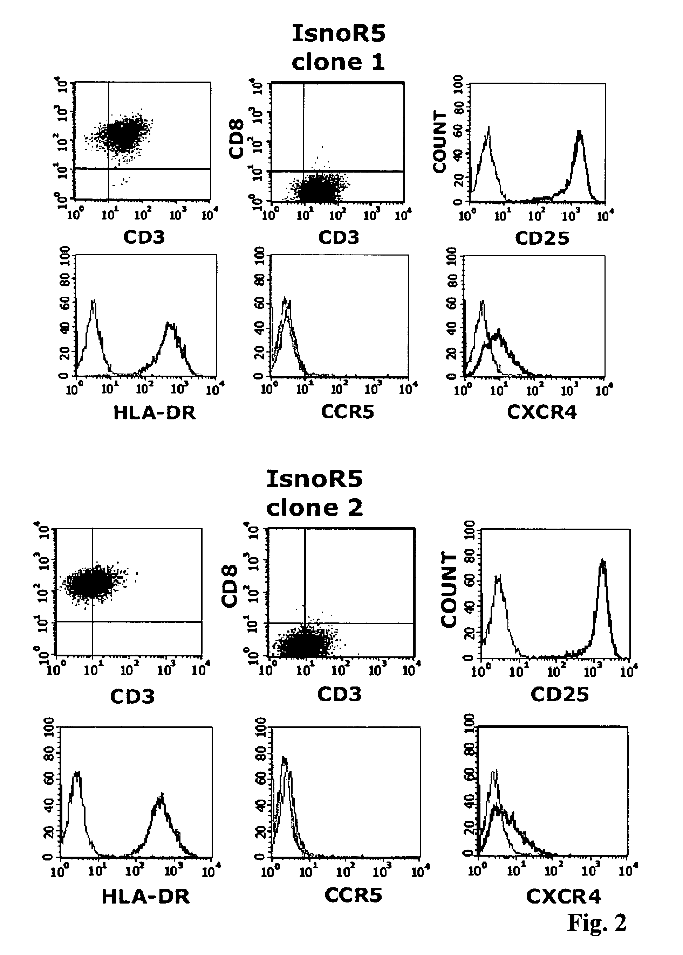

Immunophenotyping, as illustrated in figure 2, revealed IsnoR5 clones 1 and 2 to be CD4 positive T cell lines, with IsnoR5 clone 2 showing only a low expression of the CD3 surface marker. Both clones represent activated cells since they stained positive for the surface markers CD25 (IL-2 receptor α chain), and HLA-DR. Furthermore clone 1 and 2 express the chemokine receptor CXCR4 but are negative for CCR5.

### Example 2: Genotyping of the IsnoR5 clones 1 and 2

To confirm their CCR5 (Δ32/Δ32) genotype, high-molecular-weight DNA from the IsnoR5 clones 1 and 2 was subjected to PCR using primers flanking the CCR5 32 bp deletion. DNA from the human T cell lines MT-2 and PM-1, both of which are CCR5 (⁺/⁺), was included as a control. The 151 bp amplificate confirmed the IsnoR5 clones 1 and 2 to be homozygous for the CCR5 (Δ32/Δ32) genotype, while the 183 bp amplificate from MT-2 and PM-1 DNA demonstrated their CCR5 (⁺/⁺) genotype (Fig. 1A).

PCR analysis using a specific primer pair from the HTLV-I LTR confirmed the presence of HTLV-I proviral DNA (536 bp amplificate) in the IsnoR5 clones 1 and 2 and in MT-2 cells (positive control) but not in PM-1 cells (negative control) (Fig. 1B). Furthermore, expression of HTLV-I in both IsnoR5 clones was revealed by electron microscopy (data not shown).

### Example 3: Susceptibility to infection by a CXCR4-tropic HIV-1 isolate

To demonstrate susceptibility to infection 5 x 10⁵ cells each of IsnoR5 clone 1 and 2 and, as control, PM-1 cells were exposed in triplicate to 25 TCID₅₀ of the CXCR4-tropic isolate HIV-1 SF2.

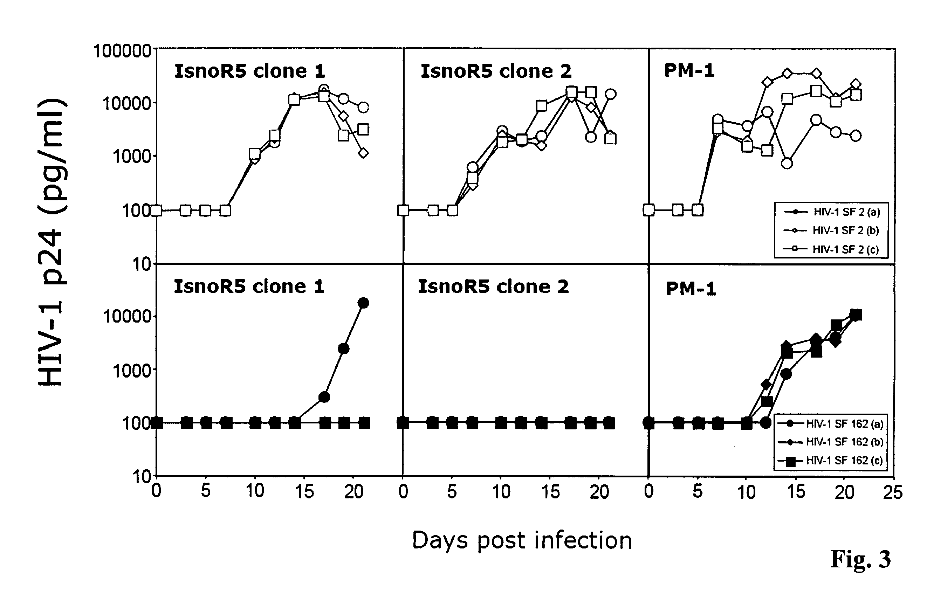

Figure 3 demonstrates similar degrees of replication of HIV-1 SF2 in all cells. With the exception of IsnoR5 clone 1, in which replication was first detected between 7 and 10 days after infection, production of virus from all cells was initially detected between days 5 and 7. These replication kinetics clearly demonstrate that clone 1 and 2 of the CCR5 negative CD4 T cell line IsnoR5 are fully susceptible to infection by an X4-tropic HIV isolate.

### Example 4: Resistance to infection by CCR5-tropic viruses and selection for coreceptor switch variants

Susceptibility to infection with a CCR5-tropic HIV-1 isolate (SF162) was tested in parallel as described above for the CXCR4-tropic SF2. Figure 3 shows that, as expected, HIV-1 SF162 replicates well on PM-1 cells but (with one surprising exception) not on the IsnoR5 clones 1 and 2. In one of the triplicate cultures of IsnoR5 clone 1, viral replication could be detected from day 17 onwards. As the V3 region of the HIV-1 gp120 surface envelope glycoprotein has a major influence on HIV-1 tropism, the corresponding region of the progeny virus was sequenced and examined for mutations. RT-PCR of viral RNA extracted from cell-free supernatant was used to amplify a fragment of gp120 env containing the V3 region of the virus, which was then cloned and sequenced. For comparison, the same was performed with RNA extracted from culture supernatant of PM-1 cells infected with HIV-1 SF162. The V3 sequence of the virus produced after infection of PM-1 cells with HIV-1 SF162 is identical (in 10 out of 10 clones) to that reported for this virus. In contrast, the progeny of HIV-1 SF162 able to grow in the IsnoR5 clone 1 have amino acid substitutions of T to Y at position 13 (T13Y), Y21H and A22T clustered around the tip of the V3 region as well as D25R and Q32K substitutions in 9 out of 9 clones analyzed. These V3 region amino acid substitutions therefore have conferred the ability to use the CXCR4 coreceptor (Hung et al., 1999). An alignment with known HIV-1 V3 sequences revealed that this 'variant' was identical to that of the CXCR4-tropic HIV-1 SF 2 isolate. The CXCR4-tropic HIV-1 SF 2 represented a minor contaminant present in the HIV-1 SF162 virus stock that expanded to dominance under the selection pressure exerted by the absence of the CCR5 chemokine coreceptor. The IsnoR5 clone 1 could therefore be useful for selecting minor CXCR4-using variants from a population dominated by CCR5-using viruses. Overall, the replication studies clearly show that the clones 1 and 2 of the CCR5 negative CD4 T cell line IsnoR5 are resistant to infection by viruses using the coreceptor CCR5.

### Example 6: A single cell line is sufficient to discriminate between use of CCR5, CXCR4 and an alternative receptor

A simple cell culture system to determine coreceptor usage was established using a panel of primary HIV-1 isolates. The TCID₅₀ of virus-containing supernatants (adjusted to approximately 5 ng/ml HIV-1 Gag p24) was determined in IsnoR5 clone 1 and clone 2 and in PBMCs from a healthy donor. These randomly selected primary isolates were derived from volunteers enrolled in a separate study and three of the isolates, P41-0, P41-9 and P-41-28, were taken at different time points from the same individual. As can be seen in Figure 4, the isolates P-7, P-26, P-41-9 and P-180 do not show any detectable replication on IsnoR5 clones 1 or 2 cells but grow readily on primary PBMC and are therefore presumed to be dependent on CCR5 as a coreceptor. In contrast, isolates P-31, P-41-0, P41-28 and P-51 replicated on both IsnoR5 clones, indicating independence from CCR5. As the isolate P-107 replicated neither on PBMC nor on IsnoR5 clones 1 and 2, it is not possible to determine its coreceptor preference.

Because HIV-1 viruses whose replication on IsnoR5 cells is inhibited by the presence of a CXCR4 selective antagonist have to use a coreceptor other than CCR5 and CXCR4, isolates P-31, P-41-0, P41-28 and P-51 were used to infect IsnoR5 clone 2 cells in the presence and absence of the bicyclam AMD 3100, a CXCR4 specific antagonist. As shown in Figure 5, all were inhibited from replication in the presence of AMD 3100. These primary isolates therefore appear to use CXCR4 as a coreceptor. This single cell culture system, IsnoR5 clone 1 or 2 in the presence and absence of AMD 3100, is sufficient to discriminate between use of CCR5, CXCR4 and an alternative receptor.

### Example 7: Comparison of biological and sequence-based methods for determining coreceptor usage

To compare the biological phenotypes obtained with our IsnoR5 cell culture system with the coreceptor usage predicted by PSSM (Jensen et al., 2003), we determined the gp120 V3 sequences for all primary isolates investigated. The PSSM-predicted coreceptor use was consistent with the results of the biological phenotyping determined using the IsnoR5 clone 2 cell culture system (Table II). The change in coreceptor preference of the viruses sequentially isolated from individual P-41 could be verified. At the first time point the isolate (P-41-0) is already CXCR4 tropic. However, nine months later the virus (P-41-9) is clearly CCR5-dependent but reverts to CXCR4 tropism after a further 19 months (P-41-28). This shows that the shift from CCR5 to CXCR4 tropic viruses is not necessarily a single event, but that reversion to CCR5 use as an intermediate can occur.

**TABLE I. Determination of coreceptor usage of primary HIV isolates by evaluating the replicative potential on IsnoR5 cell clones**

| Tropism of HIV Isolate | IsnoR5 clone 1 or 2 | |
|---|---|---|
| | minus | plus |
| | AMD3100 | AMD3100 |
| R5 | - | - |
| X4 | + | - |
| R5/X4 | + | - |
| Y | + | + |

| | | |
|---|---|---|
| Y: other than R5 and X4 | | |

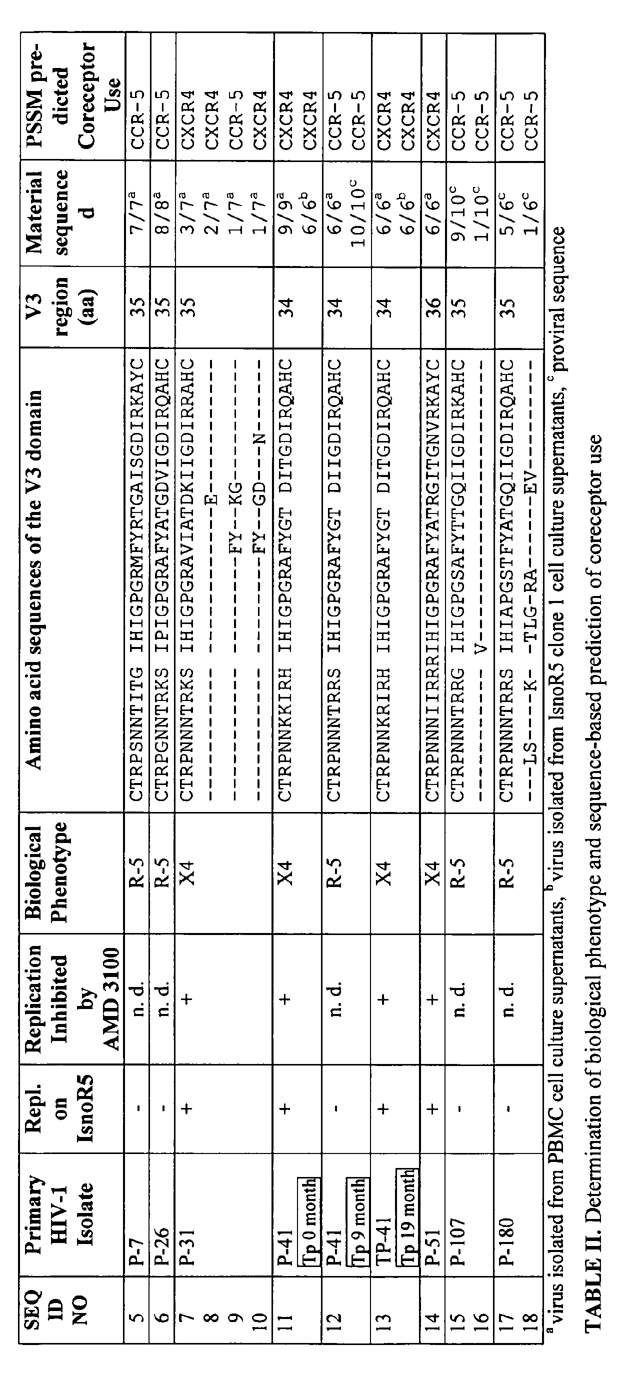

### REFERENCES

Berger EA, Murphy PM, Farber J M. 1999. Chemokine receptors as HIV coreceptors: roles in viral entry, tropism, and disease. Ann Rev Immunol 17:657-700.
Brenchley JM, Hill BJ, Ambrozak DR, Price DA, Guenaga F J, Casazza JP, Kuruppu J, Yazdani J, Migueles SA, Connors M, Roederer M, Douek DC, Koup R A. 2004. T-cell subsets that harbor human immunodeficiency virus (HIV) in vivo: implications for HIV pathogenesis. J Virol 78:1160-1168.
Briggs D, Tuttle D, Sleasman J, Goodenow M. 2000. Envelope V3 amino acid sequence predicts HIV-1 phenotype (coreceptor usage and tropism for macrophages). AIDS 14:2937-2939.
Cecilia D, Kulkarni SS, Tripathy SP, Gangakhedkar RR, Paranjape RS, Gadkari DA. 2000. Absence of coreceptor switch with disease progression in human immunodeficiency virus infections in India. Virology 271:253-258.
Cheng-Mayer C, Quiroga M, Jung JW, Dina D, Levy, J A. 1990. Viral determinants of human immunodeficiency virus type 1 T-cell or macrophage tropism, cytopathogenicity, and CD4 antigen modulation. J Virol 64:4390-4398.
Chesebro B, Wehrly K, Nishio J, Perryman S. 1996. Mapping of independent V3 envelope determinants of human immunodeficiency virus type 1 macrophage tropism and syncytium formation in lymphocytes. J Virol 70:9055-9059.
Connor RI, Sheridan KE, Ceradini D, Choe S, Landau NR. 1997. Change in coreceptor use correlates with disease progression in HIV-1-infected Individuals. J Exp Med 185:621-628.
Dean M, Carrington M, Winkler C, Huttley GA, Smith MW, Allikmets R, Goedert JJ, Buchbinder SP, Vittinghoff E, Gomperts E, Donfield S, Vlahov D, Kaslow R, Saah A, Rinaldo C, Detels R, O'Brian SJ. 1996. Genetic restriction of HIV-1 infection and progression to AIDS by a deletion allele of the CKR5 structural gene. Science 273:1856-1862.
Delobel P, Saundres-Sauné K, Casabat M, Pasquier C, Marchou B, Massip P, Izopet J. 2005. R5 to X4 switch of the predominant HIV-1 population in cellular reservoirs during effective highly active antiviral therapy. J Acquir Immune Defic Syndr 38:382-392.
Feinberg, M, Moore JP. 2002. AIDS vaccine models: challenging challenge viruses. Nat Med 8:207-210.
Fouchier RA, Groenink M, Koostra NA, Tersmette M, Huisman HG, Miedema F, Schiutemaker H. 1992. Phenotype-associated sequence variation in the third variable domain of the human immunodeficiency virus type 1 gp120 molecule. J Virol 66:3183-3187.
Gorry PR, Churchill M, Crowe SM, Cunningham AL, Gabuzda D. 2005. Pathogenesis of macrophage tropic HIV-1. Curr HIV Res 3:53-60.
Hartley O, Klasse PJ, Sattentau QJ, Moore JP. 2005. V3: HIV's Switch-Hitter. AIDS Res Hum Retrov 21:171-189.
Hoffmann NG, Seillier-Moiseiwitch F, Ahn J, Walker JM, Swanstrom R. 2002. Variability in the human immunodeficiency virus type 1 gp120 ENV protein linked to phenotypeassociated changes in the V3 loop. J Virol 76:3852-3864.
Holtkamp N, Otteken A, Findhammer S, Miller V, Kurth R, Werner A. 2000. Unexpected coreceptor usage of primary Human Immunodeficiency virus Type 1 isolates from viremic patients under highly active antiviral therapy. J Inf Di 181:513-521.
Hung CS, Vander Heyden N, Ratner L. 1999. Analysis of the critical domain in the V3 loop of human immunodeficiency virus type 1 gp120 involved in CCR5 utilization. J Virol 73:8216-8226.
Jansson M, Backstrom E, Scarlatti G, Bjorndal A, Matsuda S, Rossi P, Albert J, Wigzell H. 2001. Length variation of glycoprotein 120 V2 region in relation to biological phenotypes and coreceptor usage of primary HIV type 1 isolates. AIDS Res Hum Retroviruses 17:1405-1414.
Jekle A, Keppler OT, De Clercq E, Schols D, Weinstein M, Goldsmith MA. 2003. In vivo evolution of human immunodeficiency virus type 1 toward increased pathogenicity through CXCR4-mediated killing of uninfected CD4 T cells. J Virol 77:5846-5854.
Jensen M, Li FS, van 'Wout AB, Nickle DC, Shriner D, He HX, McLaughlin S, Shankarappa R, Margolick JB, Mullins J I. 2003. Improved coreceptor usage prediction and genotypic monitoring of R5-to-X4 transition by motif analysis of human immunodeficiency virus type 1 env V3 loop sequences. J Virol 77:13376-13388.
Johnston ER, Zijenah LS, Mutetwa S, Kantor R, Kittinunvorakoon C, Katzenstein DA. 2003. High frequency of syncytium-inducing and CXCR4-tropic viruses among human immunodeficiency virus type 1 subtype C-infected patients receiving antiretroviral treatment. J Virol 77:7682-7688.
Kitchen CMR, Philpott S, Burger H, Weiser B, Anastos K, Suchard MA. 2004. Evolution of Human Immunodeficiency Virus Type 1 Coreceptor Usage during Antiretroviral Therapy: a Bayesian Approach. J Virol 78:11296-11302.
Liu R, Paxton WA, Choe S, Ceradini D, Martin SR, Horuk R, Stuhlmann ME, Koup RA, Landau NR. 1996. Homozygous defect in HIV coreceptor accounts for resistance of some multiply-exposed HIV-1 infection. Cell 86:367-377.
Lusso P, Cocchi F, Balotta C, Markham PD, Louie A, Farci P, Pal R, Gallo RC, Reitz MS Jr. 1995. Growth of macrophage-tropic and primary human immunodeficiency virus type 1 (HIV-1) isolates in a unique CD4+ T-cell clone (PM1): Failure to downregulate CD4 and to interfere with cell-line-tropic HIV-1. J Virol 69:3712-3720.
Masciotra S, Owen SM, Rudolph D, Yang C, Wang B, Saksena N, Spira T, Dhawan S, Lal RB. 2002. Temporal relationship between V1V2 variation, macrophage replication, and coreceptor adaptation during HIV-1 disease progression. AIDS 16:1887-1898.
Meister S, Otto C, Papkalla A, Krumbiegel M, Pohlmann S, Kirchhoff F. 2001. Basic amino acid residues in the V3 loop of simian immunodeficiency virus envelope alter viral coreceptor tropism and infectivity but do not allow efficient utilization of CXCR4 as entry cofactor. Virology 284:287-296.
Miyoshi I, Kubonishi I, Yoshimoto S, Akagi T, Ohtsuki Y, Shiraishi Y, Nagata K, Hinuma Y. 1981. Type C virus particles in a cord T cell line derived by co-cultivating normal human cord leukocytes and human leukaemic T cells. Nature 294:770-771.
Pastore C, Ramos A, Mosier DE. 2004. Intrinsic obstacles to human immunodeficiency virus type 1 coreceptor switching. J Virol 78:7565-7574.
Patterson BK, Czerniewski M, Andersson J, Sullivan Y, Su F, Jiyamapa D, Burki Z, Landau A. 1999. Regulation of CCR5 and CXCR4 expression by type 1 and type 2 cytokines: CCR5 expression is downregulated by IL-10 in CD4-positive lymphocytes. Clin Immunol 91:254-262.
Penn ML, Grivel JC, Schramm B, Goldsmith MA, Margolis L. 1999. CXCR4 utilization is sufficient to trigger CD4+ T cell depletion in HIV-1 infected human lymphoid tissue. Proc Natl Aca Sci USA 96:663-668.
Resch W, Hoffman N, Swanstrom R. 2001. Improved success of phenotype prediction of the human immunodeficiency virus type 1 from envelope variable loop 3 sequence using neural networks. Virology 288:51-62.
Pillai S, Good B, Richman D, Corbeil J. 2003. A new perspective on V3 phenotype prediction. AIDS Res Hum Retroviruses 19:145-149.
Samson M, Libert F, Doranz BJ, Rucker J, Liesnard C, Farber CM, Saragosti S, Lapoumeroulie C, Cognaux J, Forceille C, Muyldermans G, Verhofstege C, Burtonboy G, Georges M, Imai T, Rana S, Yi Y, Smyth RJ, Collman RG, Doms RW, Vassart G, Parmentier M. 1996. Resistance to HIV-1 infection in Caucasian individuals bearing mutant alleles of a CCR-5 chemokine receptor gene. Nature 482:722-725.
Scarlatti G, Tresoldi E, Bjorndal A, Frederiksson R, Colognesi C, Deng HK, Malnati MS, Plebani A, Siccardi AG, Littman DR, Fenyo EM, Lusso P. 1997. In vivo evolution of HIV coreceptor usage and sensitivity to chemokine-mediated suppression. Nat Med 3:1259-1265.
Schols D. 1999. Promising anti-HIV therapeutic strategy with a small molecule CXCR4 antagonist. Verh K Acad Geneeskd Bel 61:551-564.
Seiki M, Hattori S, Hirayama Y, Yoshida M. 1983. Human adult T-cell leukaemia virus: Complete nucleotide sequence of the provirus genome integrated in leukaemia cell DNA. Proc Natl Acad Sci USA 80:3618-3622.
Shioda T, Levy JA, Cheng-Mayer C. 1992. Small amino acid changes in the V3 hypervariable region of gp120 can affect the T-cell-line and macrophage tropism of human immunodeficiency virus type 1. Proc Natl Acad Sci USA 89:9434-9438.
Vodicka MA, Goh WC, Wu II, Rogel ME, Bartz SR, Schweickart VL, Raport CJ, Emerman M. 1997. Indicator cell lines for detection of primary strains of human and simian immunodeficiency viruses. Virology 233:193-198.
Vodros D, Fenyo EM. 2005. Quantitative evaluation of HIV and SIV coreceptor use with GHOST (3) cell assay. Methods Mol Biol 304:333-342.
Xiao L, Owen SM, Goldman I, Lal AA, deJong JJ, Goudsmit J, Lal RB. 1998a. CCR5 Coreceptor Usage of Non-Syncytium-Inducing Primary HIV-1 is Independent of Phylogenetically Distinct Global HIV-1 Isolates: Delineation of Consensus Motif in the V3 Domain That Predicts CCR-5 Usage. Virology 240:83-92.
Xiao L, Rudolph DL, Owen SM, Spira TJ, Lal RB. 1998b. Adaptation to promiscuous usage of CC and CXC-chemokine co-receptors in vivo correlates with HIV-1 disease progression. AIDS 12:F137-F143.

## Claims

1. An immortalized CD4-positive cell, comprising
- a gene encoding a CXCR4 chemokine receptor that is capable of serving as a coreceptor for entry of a Human Immunodeficiency Virus Type 1 (HIV-1) into the cell, and
- a gene encoding a CCR5 receptor that is not capable of serving as a coreceptor for entry of a Human Immunodeficiency Virus Type 1 (HIV-1) into the cell.

2. The cell according to claim 1, wherein the gene encoding the CCR5 receptor bears a mutation or deletion.

3. The cell according to claim 1 or 2, wherein the gene encoding the CCR5 receptor bears a Δ32 deletion.

4. The cell according to claim 1 to 3, wherein the cell is positive for the T cell markers CD3.

5. The cell according to claim 1 to 4, wherein the cell is positive for CD25, CD45RO or HLA-DR.

6. The cell according to claims 1 to 5, wherein the cell is diploid.

7. The cell according to claim 6, wherein the cell is homozygous for the CCR5 Δ32 allele (Δ32/Δ32).

8. A cell line, tissue or organism containing a cell according to claims 1 to 7.

9. Method for producing an immortalized CD4-positive cell according to claim 1 to 7, comprising
- obtaining peripheral blood mononuclear cells (PBMC) from a donor expressing a gene encoding a CCR5 receptor that is not capable of serving as a coreceptor for entry of a Human Immunodeficiency Virus Type 1 (HIV-1) into the cell,
- isolating the CD4-positive cells from the peripheral blood mononuclear cells, and
- immortalizing the cell.

10. The method according to claim 9, wherein isolating the CD4-positive cells is performed through depleting the cells using anti-CD8 antibodies.

11. The method according to claim 9 or 10, wherein isolating the CD4-positive cells is performed through enriching the cells using anti-CD4 antibodies.

12. The method according to claims 9 to 11, wherein immortalization is performed through infection with HTLV-1, through transfection with a transforming gene of HTLV-I or through transfection of the human telomerase gene.

13. Method for the phenotypical characterization of a human immunodeficiency virus type I (HIV-1), comprising
- providing a cell that is expressing
a) a CXCR4 chemokine receptor that is capable of serving as a coreceptor for entry of an HIV-1 into the cell, and
b) a CCR5 receptor that is not capable of serving as a coreceptor for entry of an HIV-1 into the cell or no CCR5 receptor,
- adding an HIV-1 to the cell under conditions that allow for the entrance of the HIV-1 into the cell and the replication of the virus in the cell, and
- measuring the replication of the HIV-1 in the cell.

14. The method according to claim 13, further comprising comparing the replication of the HIV-1 in the cell to the replication of the HIV-1 virus in a reference cell.

15. The method according to claim 13 or 14, wherein the provided cell is a CEM 174 cell.

16. The method according to claim 13 or 14, wherein the provided cell is a cell according to claims 1 to 8.

17. The method according to claims 13 to 16, wherein the cell is provided together with a CXCR4 specific antagonist, such as bicyclam AMD 3100.

18. The method according to claim 13 to 17, wherein the measurement of the viral replication is performed using an antigen capture enzyme-linked immunosorbent assay with an antibody directed against the HIV-1 Gag p24 core protein.

19. Use of a method according to claims 9 to 18 for the diagnosis of a coreceptor change of Human Immunodeficiency Virus Type 1 (HIV-1), particularly in an individual infected with HIV-1.

20. Use of an immortalized CD4-positive cell according to claims 1 to 8 for the phenotypical characterization of a human immunodeficiency virus type I (HIV-1).

21. Use according to claim 20, wherein the phenotypical characterization is performed in a method according to claims 13 to 18.

22. Method for selecting a CXCR4-using variant of a human immunodeficiency virus type I (HIV-1) from a population of CCR5-using and CXCR4-using human immunodeficiency viruses type 1.

23. A kit for the phenotypical characterization of a human immunodeficiency virus type I (HIV-1), comprising
- a cell according to claims 1 to 8, and
- an antibody specific against the HIV-1 Gag p24 core protein.

24. The kit according to claim 23, further comprising
- a CXCR4 specific antagonist, such as bicyclam AMD 3100.
